# EUROPEAN PATENT APPLICATION

(11) **EP 4 195 139 A1**
(43) Date of publication of application: **14.06.2023**
(21) Application number: 20948867.5
(22) Date of filing: 05.08.2020
(51) Int. Cl.: G06Q 50/10, A61B 5/16, G16H 15/00, G16H 20/00

(54) **INFORMATION DISTRIBUTION SYSTEM, INFORMATION PROCESSING DEVICE, AND INFORMATION DISTRIBUTION METHOD**

(71) Applicant: Japan Tobacco Inc., Tokyo 105-6927 (JP)
(72) Inventor: OTSUKI, Shinji, Tokyo 130-8603 (JP); FUJINO, Yusuke, Tokyo 130-8603 (JP)
(74) Representative: Hoffmann Eitle
(86) International application number: PCT/JP2020/030044
(87) International publication number: WO 2022/029936

(57) **Abstract**

This information distribution system comprises a flavor suction tool, and an information processing device. The flavor suction tool includes a sensor unit which measures biometric data, and a communication unit which transmits the biometric data to an information processing device via a first network. The information processing device includes: a first communication unit which receives the biometric data measured by the flavor suction tool via the first network; an acquisition unit which acquires at least one index value based on the biometric data; a comparison unit which compares the at least one index value with a threshold value; a creation unit which creates, on the basis of the comparison result of the comparison unit, stress state information based on the at least one index value; a selection unit which selects at least one recommendation in accordance with the at least one index value; a second communication unit which transmits the stress state information created by the creation unit and the at least one recommendation selected by the selection unit, to a user terminal via a second network different from the first network.

## Description

### FIELD

The present invention relates to an information distribution system, an information processing device, and an information distribution method.

### BACKGROUND

In recent years, a technology for collecting health data by mounting a vital sensor on a device has been developed.

For example, Patent Literature 1 discloses that a vital sensor capable of measuring a heart rate or the like is mounted on a device or a case thereof, the heart rate or the like of a user is collected, and such collected health data is linked to a stress index.

Patent Literature 2 discloses that an electronic cigarette receives a state of a user in real time by a biosensor and controls an amount of substance, a substance characteristic, and a period of use of the electronic cigarette.

### CITATION LIST

### PATENT LITERATURE

[Patent Literature 1] International Publication No. 2019/175810
[Patent Literature 2] International Publication No. 2018/098371

### SUMMARY

### TECHNICAL PROBLEM

However, in Patent Literature 1, although the collected health data is associated with the stress index, the user of the device cannot take an appropriate action for stress relief according to the stress index.

In addition, in Patent Literature 2, an output for controlling the substance amount, the substance characteristic, and the period of use is performed according to the state of the user, but information for performing an appropriate action for stress relief according to a stress index is not provided.

In view of the above problem, an object of the present invention is to provide a technique that can recommend a user of a device capable of collecting predetermined data to perform an appropriate action for relieving stress.

### SOLUTION TO PROBLEM

An information distribution system according to an aspect of the present invention includes a flavor inhaler and an information processing device. The flavor inhaler includes a sensor unit configured to measure biometric data; and a communication unit configured to transmit, via a first network, the biometric data to the information processing device. The information processing device includes: a first communication unit configured to receive, via the first network, the biometric data measured by the flavor inhaler; an acquisition unit configured to acquire at least one index value based on the biometric data; a comparison unit configured to compare the at least one index value with a threshold value; a creation unit configured to create, based on a comparison result of the comparison unit, stress state information based on the at least one index value; a selection unit configured to select at least one recommendation according to the at least one index value; and a second communication unit configured to transmit, to a user terminal via a second network different from the first network, the stress state information created by the creation unit and the at least one recommendation selected by the selection unit.

An information processing device according to an aspect of the present invention includes: a first communication unit configured to receive, from a flavor inhaler via a first network, biometric data measured by the flavor inhaler; an acquisition unit configured to acquire at least one index value based on the biometric data; a comparison unit configured to compare the at least one index value with a threshold value; a creation unit configured to create, based on a comparison result of the comparison unit, stress state information based on the at least one index value; a selection unit configured to select at least one recommendation according to the at least one index value; and a second communication unit configured to transmit, to a user terminal via a second network different from the first network, the stress state information created by the creation unit and the at least one recommendation selected by the selection unit.

An information distribution method according to an aspect of the present invention is a method in an information distribution system including a flavor inhaler and an information processing device. The flavor inhaler performs: measuring biometric data; and transmitting, via a first network, the biometric data to the information processing device. The information processing device includes: receiving, via the first network, the biometric data measured by the flavor inhaler; acquiring at least one index value based on the biometric data; comparing the at least one index value with a threshold value; creating, based on a comparison result, stress state information based on the at least one index value; selecting at least one recommendation according to the at least one index value; and transmitting, to a user terminal via a second network different from the first network, the created stress state information and the selected at least one recommendation.

In the present invention, the "unit" or "device" does not simply refer to a physical means, but encompasses a case where functions equipped in the "unit" or "device" are implemented by software. The functions equipped in a single "unit" or "device" may be implemented by two or more physical means or devices, and the functions of two or more "units" or "devices" may be implemented by a single physical means or device.

### ADVANTAGEOUS EFFECTS OF INVENTION

According to the present invention, it is possible to provide a technique that can recommend a user of a device capable of collecting predetermined data to perform an appropriate action for stress relief.

### BRIEF DESCRIPTION OF THE DRAWINGS

FIG. 1 is a block diagram schematically showing a configuration example of an information distribution system according to an embodiment of the present invention.
FIG. 2 is a schematic diagram schematically showing a first configuration example of an inhaling device according to the embodiment of the present invention.
FIG. 3 is a schematic diagram schematically showing a second configuration example of an inhaling device according to the embodiment of the present invention.
FIG. 4 is a block diagram schematically showing a configuration example of a user terminal according to the embodiment of the present invention.
FIG. 5 is a block diagram schematically showing a configuration example of an information processing server according to the embodiment of the present invention.
FIG. 6 is a diagram schematically showing a first configuration example of log information DB according to the embodiment of the present invention.
FIG. 7 is a diagram schematically showing a second configuration example of the log information DB according to the embodiment of the present invention.
FIG. 8 is a diagram schematically showing a third configuration example of the log information DB according to the embodiment of the present invention.
FIG. 9 is a diagram schematically showing a configuration example of base data DB according to the embodiment of the present invention.
FIG. 10 is a flowchart showing an example of processing base data by the information processing server according to the embodiment of the present invention.
FIG. 11 is a flowchart showing an example of processing a recommendation by the information processing server according to the embodiment of the present invention.
FIG. 12 is a diagram schematically showing a display example of a display unit of the user terminal according to a first embodiment of the present invention.
FIG. 13 is a diagram schematically showing a display example of the display unit of the user terminal according to the first embodiment of the present invention.
FIG. 14 is a diagram schematically showing a display example of the display unit of the user terminal according to the first embodiment of the present invention.
FIG. 15 is a diagram schematically showing a display example of the display unit of the user terminal according to the first embodiment of the present invention.
FIG. 16 is a diagram schematically showing a display example of a display unit of the user terminal according to a first embodiment of the present invention.
FIG. 17 is a diagram schematically showing a display example of a display unit of the user terminal according to a first embodiment of the present invention.
FIG. 18 is a block diagram schematically showing a modification of a configuration of the information distribution system according to the embodiment of the present invention.

### DETAILED DESCRIPTION

Hereinafter, an embodiment will be described with reference to the accompanying drawings. In the drawings, the same constituent elements will be assigned identical reference symbols where possible, and redundant descriptions will be omitted.

### <Configuration Example of Information Distribution System>>

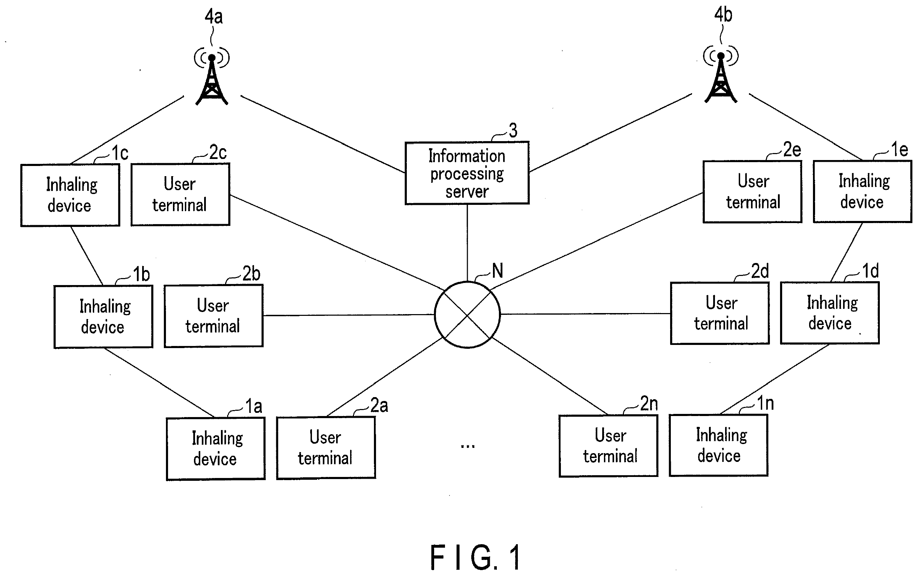

FIG. 1 is a schematic configuration diagram of an information distribution system according to the embodiment of the present invention.

As shown in FIG. 1, an information distribution system 10 includes, as an example, n inhaling devices 1a to 1n (where n is a given integral value equal to or greater than 1), n user terminal 2a to 2n (where n is a given integral value equal to or greater than 1), an information processing server 3 configured to enable communication with the user terminal 2a to 2n via a network N, and two base stations 4a and 4b. In the description that follows, when the n inhaling devices are not distinguished from one another, each one will be simply referred to as an "inhaling device 1", with part of the reference symbol omitted. Similarly, when the n user terminals are not distinguished from one another, each one of them will be simply referred to as a "user terminal 2", with part of the reference symbol omitted. It is assumed that an inhaling device 1 and a user terminal 2 to which the same alphabetical symbol is appended at the end are possessed by an identical user. For example, an inhaling device 1a and a user terminal 2a are possessed by an identical user. Similarly, when the two base stations are not distinguished from each other, each one of them will be simply referred to as a "base station 4", with part of the reference symbol omitted. The number of base stations 4 is not limited to two.

The information distribution system 10 is what is known as a "client server system". The information distribution system 10 is realized by mutual communications between the n user terminals 2, which are clients, and the information processing server 3 via the network N. The network N is realized by, for example, the Internet, a network such as a mobile communication network, a local area network (LAN), or a network that is a combination thereof.

The inhaling device 1 is an electronic device that produces a substance to be inhaled by a user. The inhaling device 1 corresponds to a flavor inhaler. A configuration example of an inhaling device that may be the inhaling device 1 will be described later. The inhaling device 1 includes a communication interface conforming to the Low Power Wide Area (LPWA) communication standard. A plurality of inhaling devices 1 construct a mesh network and can communicate with one another. The communication interface of the inhaling device 1 is not limited to the LPWA communication standard, and may be any communication interface compatible with a communication standard capable of constructing a mesh network such as a local 5G.

A user terminal 2 is a portable electronic device equipped with a communication function. For example, the user terminal 2 is a smartphone, a tablet terminal, or the like. A configuration example of the user terminal 2 will be discussed later.

The information processing server 3 is a computer equipped with an information processing function. The information processing server 3 is realized by, for example, one or more (at least one) server devices. A configuration example of the information processing server 3 will be described later. The information processing server 3 is an example of an information processing device.

The base station 4 is a base station that supports an LPWA. The base station 4 receives data from the inhaling device 1 and transmits the data to the information processing server 3. The base station 4 may be a base station that conforms to a communication standard capable of constructing a mesh network such as a local 5G.

### <<Configuration Example of Inhaling Device>>

An inhaling device is a device that produces a substance to be inhaled by the user. In the description that follows, it is assumed that the substance produced by the inhaling device is an aerosol. In another case, the substance produced by the inhaling device may be a gas.

### (1) First Configuration Example

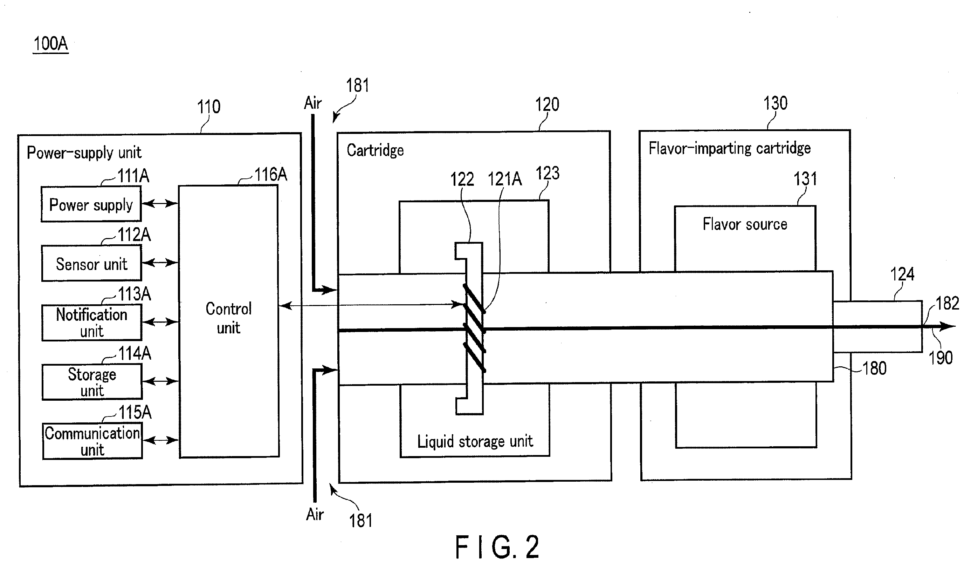

FIG. 2 is a schematic diagram schematically showing a first configuration example of the inhaling device. As shown in FIG. 2, an inhaling device 100A according to the present configuration example includes a power-supply unit 110, a cartridge 120, and a flavor-imparting cartridge 130. The power-supply unit 110 includes a power supply 111A, a sensor unit 112A, a notification unit 113A, a storage unit 114A, a communication unit 115A, and a control unit 116A. The cartridge 120 includes a heating unit 121A, a liquid guide unit 122, and a liquid storage unit 123. The flavor-imparting cartridge 130 includes a flavor source 131 and a mouthpiece 124. In the cartridge 120 and the flavor-imparting cartridge 130, an airflow path 180 is formed.

The power supply 111A accumulates electric power. Based on control by the control unit 116A, the power supply 111A supplies power to each constituent element of the inhaling device 100A. The power supply 111A may be configured of, for example, a chargeable battery such as a lithium-ion secondary battery.

The sensor unit 112A acquires various types of information relating to the inhaling device 100A. As an example, the sensor unit 112A is configured of a vital sensor that measures biometric data, a pressure sensor such as a microphone capacitor, a flowrate sensor, a temperature sensor, etc., and acquires values involved with user inhalation. As another example, the sensor unit 112A is configured of an input device which receives an information input from the user, such as a button, a switch, etc.

The notification unit 113A notifies the user of the information. The notification unit 113A is configured of, for example, a light-emitting device which emits light, a display device which displays an image, a sound output device which outputs sound, a vibration device which vibrates, etc.

The storage unit 114A stores various types of information for operation of the inhaling device 100A. The storage unit 114A is configured of, for example, a non-volatile storage medium such as a flash memory.

The communication unit 115A is a communication interface that enables communications compatible with the LPWA communication standard to be performed. The communication unit 115A transmits the biometric data to the information processing server 3 via a first network. The first network is a mesh network including the plurality of inhaling devices 1. The standard of the communication unit 115A is not limited to the LPWA communication standard, and may be anything compatible with a communication standard capable of constructing a mesh network such as a local 5G.

The control unit 116A functions as an arithmetic processor and a controller, and controls the entire operation of the inhaling device 100A in accordance with various programs. The control unit 116A is realized by, for example, electronic circuitry such as a central processing unit (CPU), or a microprocessor.

The liquid storage unit 123 stores an aerosol source. By atomizing the aerosol source, aerosol is produced. The aerosol source is, for example, a polyhydric alcohol such as glycerin and propylene glycol, and a liquid such as water. The aerosol source may contain a tobacco-derived or non-tobacco-derived flavor component. In a case where the inhaling device 100A is a medical aspirator such as a nebulizer, the aerosol source may contain a drug.

The liquid guide unit 122 guides the aerosol source which is a liquid stored in the liquid storage unit 123 from the liquid storage unit 123, and holds the aerosol source. The liquid guide unit 122 is, for example, a wick formed by twisting a fiber material such as glass fiber or a porous material such as a porous ceramic. In this case, the aerosol source stored in the liquid storage unit 123 is guided by the capillary effect of the wick.

The heating unit 121A heats the aerosol source, atomizes the aerosol source and produce aerosol. In the example shown in FIG. 2, the heating unit 121A is configured as a coil, and is wound around the liquid guide unit 122. When the heating unit 121A generates heat, the aerosol source held in the liquid guide unit 122 is heated and atomized, thus producing aerosol. The heating unit 121A generates heat when fed from the power supply 111A. As an example, feeding may be performed when the sensor unit 112A has detected the commencement of inhaling by the user and/or the input of predetermined information. Feeding may be stopped when the sensor unit 112A has detected the termination of inhaling by the user and/or the input of predetermined information.

The flavor source 131 is a constituent element for imparting a flavor component to aerosol. The flavor source 131 may contain a tobacco-derived or non-tobacco-derived flavor component.

The airflow path 180 is a path of air inhaled by the user. The airflow path 180 has a tubular structure including, at one of both ends, an air inflow hole 181, which is an inlet of air flowing to the airflow path 180, or an air outflow hole 182, which is an outlet of air flowing from the airflow path 180. In the middle of the airflow path 180, the liquid guide unit 122 is arranged on an upstream side (the side closer to the air inflow hole 181), and a flavor source 131 is arranged on a downstream side (the side closer to the air outflow hole 182). The air flowing in from the air inflow hole 181 in accordance with user inhalation is mixed with the aerosol produced by the heating unit 121A, and, as shown by an arrow 190, passes through the flavor source 131 and is transported to the air outflow hole 182. When a mixed fluid of the aerosol and air passes through the flavor source 131, a flavor component included in the flavor source 131 is imparted to the aerosol.

The mouthpiece 124 is a member which comes in contact with the user's mouth during inhaling. In the mouthpiece 124, the air outflow hole 182 is arranged. The user can introduce a mixed fluid of the aerosol and air into the oral cavity through inhalation via the mouthpiece 124 put in the user's mouth.

A configuration example of the inhaling device 100A has been described above. As a matter of course, the configuration of the inhaling device 100A is not limited to the above, and various configurations that will be described below as examples may be adopted.

As an example, the inhaling device 100A may not include a flavor-imparting cartridge 130. In this case, a mouthpiece 124 is provided in the cartridge 120.

As another example, the inhaling device 100A may include a plurality of types of aerosol sources. Still other types of aerosol may be produced by a chemical reaction caused by a plurality of types of aerosol produced by a plurality of types of aerosol sources being mixed in the airflow path 180.

Moreover, the means for atomizing the aerosol source are not limited to heating by the heating unit 121A. For example, the aerosol source may be atomized by means such as vibration atomizing or induction heating.

### (2) Second Configuration Example

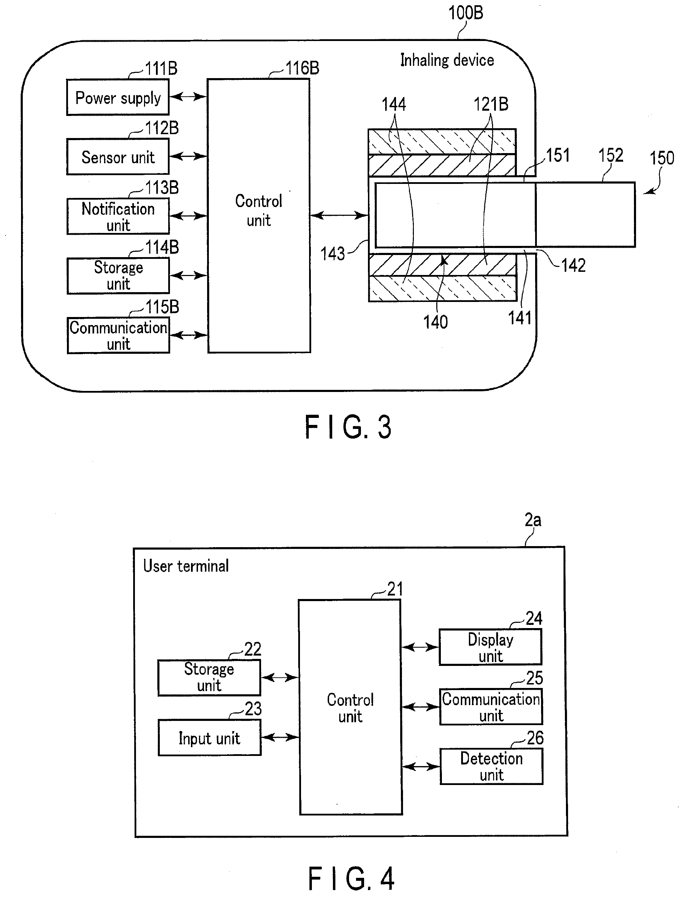

FIG. 3 is a schematic diagram schematically showing a second configuration example of the inhaling device. As shown in FIG. 3, an inhaling device 100B according to the present configuration example includes a power supply 111B, a sensor unit 112B, a notification unit 113B, a storage unit 114B, a communication unit 115B, a control unit 116B, a heating unit 121B, a holding part 140, and a heat-insulating part 144.

The power supply 111B, the sensor unit 112B, the notification unit 113B, the storage unit 114B, the communication unit 115B, and the control unit 116B are substantially identical to the corresponding constituent elements included in the inhaling device 100A according to the first configuration example.

The holding part 140 includes an internal space 141, and holds a stick-shaped base material 150 with part of the stick-shaped base material 150 housed in the internal space 141. The holding part 140 includes an opening 142 which allows the internal space 141 to be communicated to the outside, and holds the stick-shaped base material 150 inserted into the internal space 141 from the opening 142. The holding part 140 is, for example, a tubular body including the opening 142 and a bottom portion 143 as bottom surfaces, and defines a columnar internal space 141. The holding part 140 is also equipped with a function of defining a path of air that is supplied to the stick-shaped base material 150. An air inflow hole which is an inlet of air to such a path is arranged in, for example, the bottom portion 143. On the other hand, an air outflow hole which is an outlet of air from such a path is the opening 142.

The stick-shaped base material 150 includes a base material portion 151 and a suction portion 152. The base material portion 151 includes an aerosol source. In the present configuration example, the aerosol source is not limited to a liquid, and may be a solid. With the stick-shaped base material 150 held in the holding part 140, at least part of the base material portion 151 is housed in the internal space 141, and at least part of the suction portion 152 projects from the opening 142. Through user inhalation via the suction portion 152 projecting from the opening 142 which comes into contact with the user's mouth, air flows in from an air inflow hole (not illustrated) to the internal space 141, and reaches the user's oral cavity together with the aerosol produced from the base material portion 151.

The heating unit 121B has a configuration similar to that of the heating unit 121A according to the first configuration example. However, in the example shown in FIG. 3, the heating unit 121B is configured in a film shape, and is arranged so as to cover the outer periphery of the holding part 140. When the heating unit 121B generates heat, the base material portion 151 of the stick-shaped base material 150 is heated from the outer periphery, and thereby aerosol is produced.

The heat-insulating part 144 prevents heat transfer from the heating unit 121B to another constituent element. For example, the heat-insulating part 144 is configured from a vacuum-insulating material, an aerogel-insulating material, etc.

A configuration example of the inhaling device 100B has been described above. As a matter of course, the configuration of the inhaling device 100B is not limited to the above, and various configurations described below as examples may be adopted.

As an example, the heating unit 121B may be configured in a blade shape and arranged so as to project from the bottom portion 143 of the holding part 140 into the internal space 141. In this case, the blade-shaped heating unit 121B is inserted into the base material portion 151 of the stick-shaped base material 150, and the base material portion 151 of the stick-shaped base material 150 is heated from the inside. As another example, the heating unit 121B may be arranged so as to cover the bottom portion 143 of the holding part 140. Also, the heating unit 121B may be configured as a combination of two or more of a first heating unit that covers an outer periphery of the holding part 140, a blade-shaped second heating unit, and a third heating unit that covers a bottom portion 143 of the holding part 140.

As another example, the holding part 140 may include an open/close mechanism such as a hinge, which opens and closes part of an outer shell that forms the internal space 141. By opening and closing the outer shell, the holding part 140 may hold the stick-shaped base material 150 inserted into the internal space 141. In this case, the heating unit 121B may be provided in the place of the holding part 140 which holds the stick-shaped base material 150, and may heat the stick-shaped base material 150 while pressing it

Moreover, the means for atomizing the aerosol source are not limited to heating by the heating unit 121B. For example, the means for atomizing the aerosol source may be based on induction heating.

Also, the inhaling device 100B may further include the heating unit 121A, the liquid guide unit 122, the liquid storage unit 123, and the airflow path 180 according to the first configuration example, and the air outflow hole 182 of the airflow path 180 may also function as an air inflow hole to the internal space 141. In this case, a mixed fluid of the aerosol and air produced by the heating unit 121A flows into the internal space 141, is further mixed with the aerosol produced by the heating unit 121B, and reaches the oral cavity of the user.

The inhaling device 1 configured as described above begins measurement of biometric data based on activation, and transmits the measured biometric data to the information processing server 3 via the first network. In an example, the inhaling device 1 begins measurement of the biometric data based on the activation of the inhaling device 1 in response to inhalation of the inhaling device 1 by the user. In another example, the inhaling device 1 begins measurement of the biometric data based on an input of the activation by the user using a button, a switch, or the like of the inhaling device 1.

Here, an example of biometric data measured by the inhaling device 1 will be described. The biometric data is data that can be measured in the inhaling device 1 using a known technique with respect to indexes, such as pulses, perspiration, salivary components, heartbeats, respiration, words, and a grip strength. The data relating to salivary components may include data such as concentrations relating to amylase (u/L), serotonin (µg/mL), cotisol (Pmol/mL), nitric oxide metabolite (µg/g) (salivary nitrate ion). The measurement data related to salivary components may include data such as volatile sulfur compounds (ppb) (hydrogen sulfide, methyl mercaptan, and dimethyl sulfide), acidity (pH), buffer capacity (pH), white blood cells (cells/µL), protein (mg/mL), ammonia (µg/mL), and viscosity (cp) in addition to the above-mentioned data. The data related to respiration may include a puff amount, a puff interval, a time until expiration, and the like. The biometric data is not limited to the above, and may be any biometric data of an index related to measurement of stress. The biometric data may be measured at any timing such as before smoking (before use of the inhaling device 1) and after smoking (after use of the inhaling device 1) as well as at a timing while the user is smoking through use of the inhaling device 1 (during use of the inhaling device 1).

### <<Configuration Example of User Terminal>>

FIG. 4 is a block diagram schematically showing a configuration example of a user terminal according to an embodiment of the present invention.

The user terminal 2a includes a control unit 21, a storage unit 22, an input unit 23, a display unit 24, a communication unit 25, and a detection unit 26. These units are mutually connected via a bus line.

The control unit 21 controls the entire operation of the user terminal 2a in accordance with a program stored in the storage unit 22. The control unit 21 is configured of, for example, electronic circuitry such as a processor. For example, the processor is a CPU or the like.

The storage unit 22 is configured of a main storage and an auxiliary storage. The main storage is configured of, for example, a volatile memory that provides a work area for the processor. The main storage is configured of, for example, a random access memory (RAM) or the like. The auxiliary storage is configured of, for example, a non-volatile memory that stores various types of information and programs for operation of the user terminal 2a. The auxiliary storage is configured of, for example, a hard disk drive (HDD), a solid-state drive (SSD), or the like.

Based on the user's operation, the input unit 23 receives an instruction. The input unit 23 is configured of, for example, a keyboard, a touchpad, or the like.

The display unit 24 displays a variety of screens. The display unit 24 is configured of, for example, a liquid crystal display.

The communication unit 25 is a communication interface that enables communications compatible with a given communication standard to be performed. The communication unit 25 enables communication with the information processing server 3 via a second network different from the first network. The second network is the network N described above.

The detection unit 26 is configured of a sensor that detects various types of information. The detection unit 26 includes a sensor that detects position information on the user terminal 2a. For example, the position information is information indicating latitude and longitude. For example, the sensor that detects the position information is a global positioning system (GPS) sensor.

The above-described user terminal 2a transmits user data, to be described below as an example, to the information processing server 3. For example, the user data includes position information on the user terminal 2a. The user data may include, in addition to the position information, time information related to acquisition of the position information. The time information related to the acquisition of the position information is information indicating the time at which the user terminal 2a acquired the position information.

First, the user terminal 2a continuously or intermittently acquires position information on the user terminal 2a based on the activation of a specific application. The user terminal 2a acquires position information on the user terminal 2a detected by the detection unit 26. The position information on the user terminal 2a corresponds to position information on the user who possesses the user terminal 2a and position information on the inhaling device 1a owned by the user. Based on the acquisition of the position information, the user terminal 2a acquires time information related to the acquisition of the position information. The user terminal 2a accordingly stores the position information in the storage unit 22 in association with the time information related to the acquisition of the position information.

Next, the user terminal 2a transmits the user data, including at least the position information, to the information processing server 3 via the communication unit 25. The user terminal 2a may transmit the user data to the information processing server 3 in real time in response to the acquisition of the position information.

Although an example in which the user terminal 2a acquires the position information on the user terminal 2a based on the activation of a specific application has been described, the embodiment is not limited thereto. The user terminal 2a may acquire the position information continuously or intermittently regardless of activation of a specific application.

The user data may include user information of the user who possesses the user terminal 2a. The user information is information unique to the user who possesses the user terminal 2a. For example, the user information is a preset user ID, user attribute information, user preference information, and the like. The user ID is an identifier uniquely assigned to the user terminal 2. The attribute information is information indicating user attributes, such as gender and age. The preference information is information indicating user preferences, such as those relating to taste. The attribute information and the preference information are input from the input unit 23 by the user. The preference information may be suitably updated. The user information is stored in the storage unit 22.

The user data may include type information indicating the type of the inhaling device 1a. The type information is, for example, information such as the model number of the inhaling device which indicates the difference in heating temperature. The type information is stored in the storage unit 22. The user data may include other information.

A hardware configuration of the user terminal 2a is not limited to the above-described configuration. The above-described constituent elements of the user terminal 2a may be suitably omitted or changed, and a new constituent element may be added to the user terminal 2a. Herein, a configuration example of the user terminal 2a has been described; however, the configuration of the user terminal 2 other than the user terminal 2a is similar to that of the user terminal 2a, and the description thereof will be omitted. Similarly to the user terminal 2a, the user terminal 2 other than the user terminal 2a may transmit user data of the user terminal 2 to the information processing server 3.

### <<Configuration Example of Information Processing Server>>

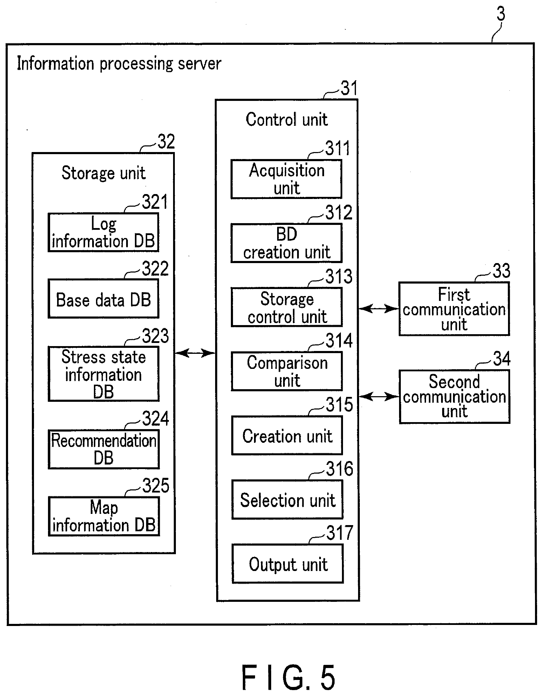

FIG. 5 is a block diagram schematically showing a configuration example of an information processing server according to an embodiment of the present invention.

The information processing server 3 includes a control unit 31, a storage unit 32, and a first communication unit 33, and a second communication unit 34. These units are mutually connected via a bus line.

The control unit 31 controls the entire operation of the information processing server 3 in accordance with programs stored in the storage unit 32. The control unit 31 is configured of, for example, electronic circuitry such as a processor. The processor is, for example, a CPU or the like. By executing the programs stored in the storage unit 32, the control unit 31 realizes the respective units that will be discussed later, and executes a variety of operations.

The storage unit 32 is configured of a main storage and an auxiliary storage, similarly to the above-described storage unit 22. The storage unit 32 stores, in the control unit 31, programs for causing the control unit 31 to realize the respective units that will be discussed later. The storage unit 32 stores log information DB 321, base data DB 322, stress state information DB 323, recommendation DB 324, and map information DB 325.

The log information DB 321 is a database that records log information mainly based on biometric data transmitted from the inhaling device 1 to the information processing server 3 for each predetermined period in a time series. The log information includes a value of each index (hereinafter also referred to as an "index value") obtained based on the biometric data. The index value is a value per unit obtained based on the biometric data of each index. For example, the index value related to the pulse is a pulse rate per minute obtained based on the biometric data on the pulse. The index value is obtained by the information processing server 3 based on the biometric data. The log information DB321 is updated every time the information processing server 3 receives the biometric data from the inhaling device 1. A configuration example of the log information DB 321 will be discussed later.

The base data DB 322 is a database that records base data created based on the log information accumulated in the log information DB 321. The base data includes a plurality of statistical values such as an average value, a maximum value, and a minimum value of the index values included in the log information. The plurality of statistical values may include a statistical value of each user associated with each user ID, a statistical value of each group obtained by grouping a plurality of users according to an attribute, a statistical value of all users within the plurality of users, and the like. Each statistical value may be a threshold value for determining the user stress state. A configuration example of the base data DB 322 will be discussed later.

The stress state information DB 323 is a database that records stress state information indicating a user stress state. The stress state information is information indicating, as a stress state, a result of comparing at least one index value of the user with a threshold value. The result of comparing the at least one index value of the user with the threshold value includes a result of comparing a stress level based on the at least one index value of the user with a stress level based on a statistical value serving as a threshold value. The stress level is a level of stress obtained by quantifying the index value and the statistical value according to a predetermined rule. The biometric data on which the index value and the statistical value are based correlates with the stress level. Therefore, the index value and the statistical value may be converted into a stress level. For example, the stress state information is text indicating a stress state such as "high stress", a graph indicating a stress state in an identifiable manner, or the like, but is not limited thereto, and may be in any format. For example, the mode in which the stress state can be identified is a mode in which an index value and a threshold value are clearly indicated. In this example, the stress state can be identified by a degree of divergence between the index value and the threshold value. For example, the mode in which the stress state can be identified is a mode in which a stress level based on an index value and a stress level based on a statistical value serving as a threshold value are clearly indicated. In this example, the stress state can be identified by the degree of divergence between the stress level based on the index value and the stress level based on the statistical value serving as the threshold value.

The recommendation DB 324 is a database that records recommendation information (hereinafter also referred to as "recommendation") to be provided to the user.

In an example, the recommendation is a recommendation based on stress state information. The recommendation based on the stress state information includes a message that recommends an action to reduce stress. For example, the recommendation based on the stress state information includes a message such as "Take a break for 5 minutes" or "XX is recommended". The content of the message may be suitably updated.

In another example, the recommendation is a recommendation based on information differing from the stress state information. For example, the recommendation based on the information differing from the stress state information is a recommendation based on the position information on the user. The recommendation based on the position information on the user is a rest facility near the current location of the user. The rest facility is a facility where the user can take a rest, such as a coffee shop, a cafe, or a bookstore. Information on the rest facility may be stored either in the map information DB 325 or in the storage unit 32 in advance. The information on the rest facility may be downloaded from a server (not shown) to the information processing server 3 via the network N. The information on the rest facility may be suitably updated. The recommendation based on the information differing from the stress state information is not limited to the recommendation based on the user's position information. An example recommendation will be described later.

The map information DB 325 is a database storing map information related to an existing general map indicating states of items distributed on the ground. The map information DB 325 may include position information on each facility. The map information DB 325 may include attribute information on each facility. The attribute information is information indicating a category (bookstore, coffee shop, or the like) on each facility or an item (book, coffee, or the like) handled by each facility. The map information DB 325 may be stored in advance in the storage unit 32, or may be downloaded from a server (not illustrated) to the information processing server 3 via the network N. The map information DB 325 may be suitably updated.

The first communication unit 33 is a communication interface that enables communications compatible with a given communication standard of the first network. The first communication unit 33 enables communications between the information processing server 3 and the inhaling device 1 via the first network.

The second communication unit 34 is a communication interface that enables communications compatible with a given communication standard of the second network. The second communication unit 34 enables communications between the information processing server 3 and the user terminal 2 via the second network.

A hardware configuration of the information processing server 3 is not limited to the above-described configuration. The above-described constituent elements of the information processing server 3 may be suitably omitted or changed, and a new constituent element may be added to the information processing server 3.

Each of the units realized by the above-described control unit 31 will be described.

The control unit 31 realizes an acquisition unit 311, a base data creation unit 312, a storage control unit 313, a comparison unit 314, a creation unit 315, a selection unit 316, and an output unit 317.

Based on the activation of the inhaling device 1, the acquisition unit 311 receives the biometric data measured by the inhaling device 1 via the first communication unit 33. The acquisition unit 311 acquires at least one index value based on biometric data. The acquisition unit 311 acquires user data from each of the plurality of user terminals 2 via the second communication unit 34.

The base data creation unit 312 creates base data using log information mainly based on the biometric data acquired by the acquisition unit 311. Hereinafter, the base data creation unit 312 is also referred to as a "BD creation unit 312". A configuration example of the base data will be discussed later.

The storage control unit 313 performs control so that the base data created by the BD creation unit 312 is stored in the base data DB 322.

The comparison unit 314 compares at least one index value based on the biometric data acquired by the acquisition unit 311 with a threshold value based on the statistical value included in the base data. For example, in a case where the index value is a pulse rate, the comparison unit 314 compares the pulse rate of the user with a threshold value corresponding to an average value or the like of the pulse rates of the user included in the base data. Instead, the comparison unit 314 compares the pulse rate of the user with a threshold value corresponding to an average value or the like of pulse rates of an attribute group (for example, persons in their 30s). Instead, the comparison unit 314 compares the pulse rate of the user with a threshold value corresponding to an average value or the like of all pulse rates.

The creation unit 315 creates stress state information based on at least one index value, in turn based on the comparison result of the comparison unit 314. For example, in a case where the index value is the pulse rate of the user, the creation unit 315 determines the stress state based on the pulse rate of the user and creates the stress state information.

The selection unit 316 selects at least one recommendation in accordance with at least one index value.

The output unit 317 outputs the stress state information created by the creation unit 315 to the user terminal 2 via the second communication unit 34. The output unit 317 outputs at least one recommendation selected by the selection unit 316 to the user terminal 2 via the second communication unit 34. The output unit 317 transmits a control command for controlling the inhaling device 1 in accordance with at least one index value via the second communication unit 34. For example, the control command is a command for controlling a heating temperature, a smoke amount, and the like of the inhaling device 1.

### <<Configuration Example of Log Information DB>>

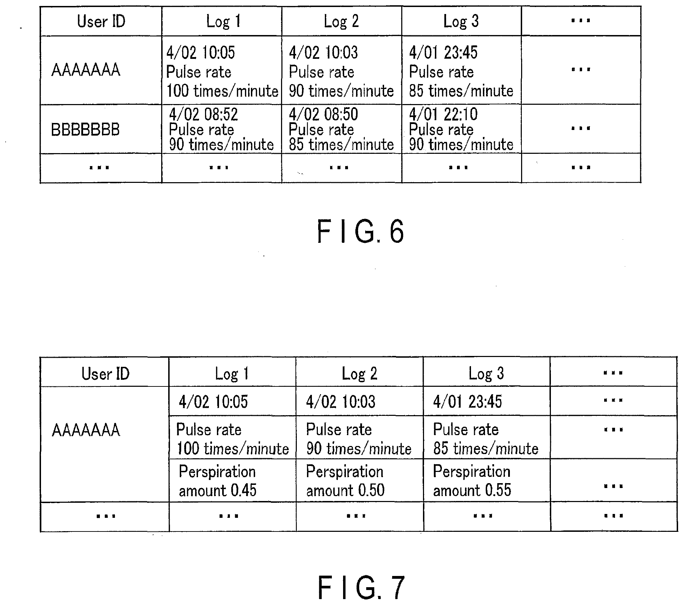

FIG. 6 is a diagram schematically showing a first configuration example of log information DB according to the embodiment of the present invention.

The log information DB 321 includes, for example, a record in which a "user ID" and log information for each predetermined period in a time series are associated with each other. Data that configures a record is configured based on the biometric data and the user data. The "user ID" corresponds to the user ID included in the user information of the user data. The log information includes "time" and an "index value". The "time" corresponds to the time at which the information processing server 3 acquires the biometric data from the inhaling device 1. The time at which the information processing server 3 acquires the biometric data corresponds to time at which the inhaling device 1 measures the biometric data. The "index value" corresponds to an index value obtained based on biometric data. In FIG. 6, the "index value" is, for example, a pulse rate, which is a value such as "pulse rate 100 times/minute". The data that configures the record may include the user attribute information and the user preference information included in the user information of the user data. The log information DB 321 may include records of a plurality of users. The log information DB 321 may be updated in response to the addition of log information.

FIG. 7 is a diagram schematically showing a second configuration example of the log information DB according to the embodiment of the present invention.

The log information DB 321 includes, for example, a record in which a "user ID" and log information for each predetermined period in a time series are associated with each other. Data that configures a record is configured based on the biometric data and the user data. The "user ID" corresponds to the user ID included in the user information of the user data. The log information includes "time" and an "index value". The "time" corresponds to the time at which the information processing server 3 acquires the biometric data from the inhaling device 1. The "index value" corresponds to an index value obtained based on biometric data. In FIG. 7, the log information may include a plurality of index values. The "index value" includes, for example, a pulse rate and a perspiration amount, and are values such as "pulse rate 100 times/min", "perspiration amount 0.45", or the like. The data that configures the record may include the user attribute information and the user preference information included in the user information of the user data. The log information DB 321 may include records of a plurality of users. The log information DB 321 may be updated in response to the addition of log information.

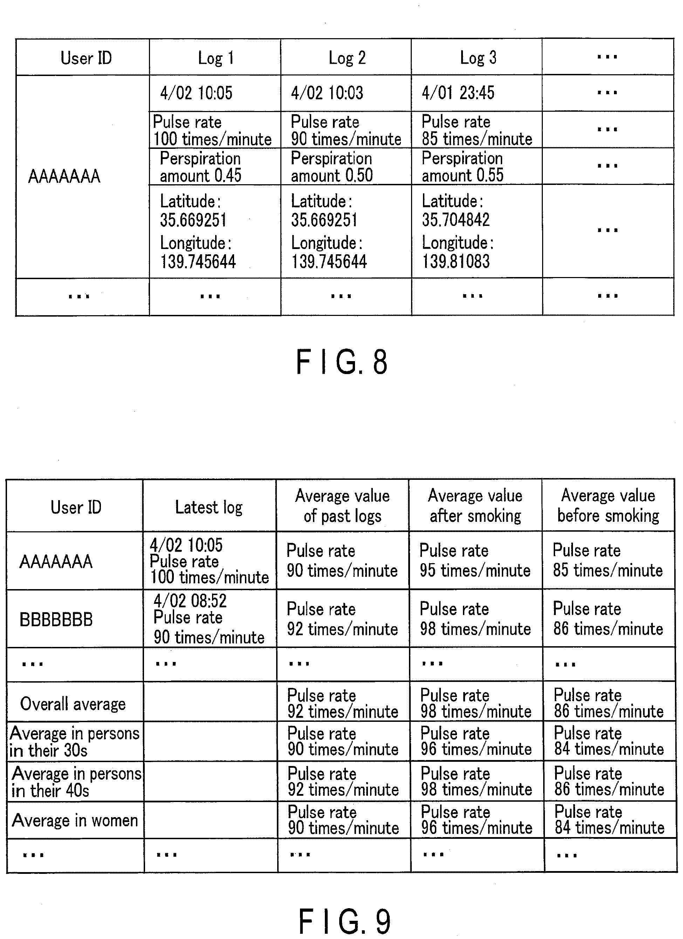

FIG. 8 is a diagram schematically showing a third configuration example of the log information DB according to the embodiment of the present invention.

The log information DB 321 includes, for example, a record in which a "user ID" and log information for each predetermined period in a time series are associated with each other. Data that configures a record is configured based on the biometric data and the user data. The "user ID" corresponds to the user ID included in the user information of the user data. The log information includes "time", an "index value", and a "position". The "time" corresponds to the time at which the information processing server 3 acquires the biometric data from the inhaling device 1. The "index value" corresponds to an index value obtained based on biometric data. In FIG. 8, the log information may include a plurality of index values. The "index value" includes, for example, a pulse rate and a perspiration amount, and are values such as "pulse rate 100 times/min", "perspiration amount 0.45", or the like. The "position" corresponds to position information included in the user data. As exemplified below, the information processing server 3 stores, in the log information, the position information included in the user data. The information processing server 3 compares the time specified by the time information related to the acquisition of the position information included in the user data with the "time" in the log information. The information processing server 3 detects the log information storing the "time" that either coincides or substantially coincides with the time specified by the time information related to the acquisition of the position information within a predetermined range. The information processing server 3 stores, in the detected log information, the position information associated with the time information related to the acquisition of the position information. The data that configures the record may include the user attribute information and the user preference information included in the user information of the user data. The log information DB 321 may include records of a plurality of users. The log information DB 321 may be updated in response to the addition of log information.

FIG. 9 is a diagram schematically showing a configuration example of base data DB according to the embodiment of the present invention.

A "latest log" is shown for convenience and to be compared with each statistical value included in the base data; it is not included in the base data DB 322. The base data DB 322 includes, for example, a record in which a "user ID" is associated with an "average value of past logs", an "average value after smoking", an "average value before smoking", and the like. Data that configures a record is configured based on base data. The "user ID" corresponds to the user ID included in the user information of the user data. The "average value of past logs" corresponds to a statistical value based on an average of index values included in past log information for each user. The "average value after smoking" corresponds to a statistical value based on an average of index values after smoking included in the past log information for each user. The "average value before smoking" corresponds to a statistical value based on an average of index values before smoking included in the past log information for each user. The statistical value based on the average of the index values after smoking is a statistical value based on the average of the index values based on the biometric data acquired at the end time when the user ceases use of the inhaling device 1 The statistical value based on the average of the index values before smoking is a statistical value based on the average of the index values based on the biometric data acquired at the start time when the user commences use of the inhaling device 1. The base data DB 322 may include statistical values such as maximum values and minimum values after smoking in addition to the average value after smoking. The base data DB 322 may include statistical values such as maximum values and minimum values before smoking in addition to the average value before smoking. The base data DB 322 may include statistical values such as average values, maximum values, and minimum values of the amount of change before and after smoking. In a case where the base data DB 322 includes base data of a plurality of users, an "average value of past logs", an "average value after smoking", and an "average value before smoking" may include a statistical value for all users within the plurality of users and a statistical value of each group obtained by grouping the plurality of users according to an attribute. For example, the base data DB 322 may include statistical values in which items such as an "overall average", an "average in persons in their 30s", an "average in persons in their 40s", and an "average in women" are each associated with an "average value of past logs", an "average value after smoking", and an "average value before smoking". In FIG. 9, the statistical value indicates the pulse rate, but is not limited thereto, and may be the perspiration amount or the like. The base data DB 322 may include statistical values of a plurality of indexes.

### <<Example of Processing by Information Processing Server>>

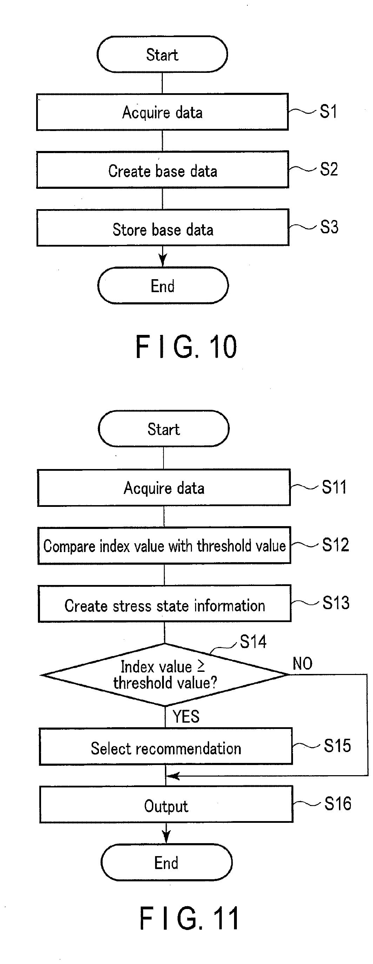

FIG. 10 is a flowchart showing an example of processing base data by the information processing server according to the embodiment of the present invention.

The processing procedure described below is merely an example, and each act of processing may be changed as much as possibility allows. In addition, in the processing procedure described below, steps can be omitted, replaced, and added as appropriate according to the embodiment.

The acquisition unit 311 acquires various data (step S1). In step S1, for example, the acquisition unit 311 acquires, via the first communication unit 33, biometric data respectively measured by the inhaling devices 1 based on activation of the respective inhaling devices 1. Here, the first communication unit 33 receives the biometric data respectively measured by the plurality of inhaling devices 1 via the first network. The acquisition unit 311 acquires one or more index values based on the biometric data, and acquires the one or more index values at predetermined time intervals. The acquisition unit 311 sequentially adds the log information including one or more index values to the log information DB 321 every time the predetermined period has passed in a time series. The acquisition unit 311 stores, in the log information, the time at which the biometric data is acquired. The acquisition unit 311 acquires various types of information included in the user data from each of the plurality of user terminals 2 via the second communication unit 34. For example, the acquisition unit 311 acquires the position information from each of the plurality of user terminals 2 via the second communication unit 34. Here, the second communication unit 34 receives the position information from each of the plurality of user terminals 2 via the second network. For example, the acquisition unit 311 acquires the user information from each of the plurality of user terminals 2 via the second communication unit 34. Here, the second communication unit 34 receives the user information from each of the plurality of user terminals 2 via the second network. The acquisition unit 311 may separately acquire, from each of the plurality of user terminals 2, various types of information described as may be included in the user data. The acquisition unit 311 accordingly adds the position information, the user information, and the like to the log information DB 321 as needed.

The BD creation unit 312 creates the base data based on the log information stored in the log information DB 321 (step S2). In step S2, for example, the BD creation unit 312 calculates a statistical value for each user. In this example, the BD creation unit 312 calculates a statistical value such as an average value, a maximum value, or a minimum value for at least one index value associated with the user ID of each user terminal 2 included in the log information. The BD creation unit 312 may calculate a statistical value for all users within the plurality of users. The BD creation unit 312 may calculate a statistical value for each group in which a plurality of users are grouped according to an attribute.

The BD creation unit 312 may calculate a statistical value for each user, a statistical value for all users within a plurality of users, and a statistical value for each group obtained by grouping a plurality of users according to an attribute under various conditions exemplified below. For example, the BD creation unit 312 calculates a statistical value such as an average value, a maximum value, or a minimum value for each time zone. In this example, the BD creation unit 312 uses the time information included in the log information. For example, the BD creation unit 312 calculates a statistical value such as an average value, a maximum value, or a minimum value after smoking or before smoking. In this example, the BD creation unit 312 uses the time information included in the log information. The BD creation unit 312 may acquire, as the log information after smoking, the last log information among a collection of log information that continues for a certain period of time. The BD creation unit 312 may acquire, as the log information before smoking, the first log information among a collection of log information that continues for a certain period of time. For example, the BD creation unit 312 calculates a statistical value such as an average value, a maximum value, or a minimum value of the amount of change before and after smoking. In this example, the BD creation unit 312 calculates the statistical value of the amount of change before and after smoking using the statistical value before smoking and the statistical value after smoking. For example, the BD creation unit 312 calculates a statistical value such as an average value, a maximum value, or a minimum value of each user at a certain time point in a given day. In this example, the BD creation unit 312 uses the time information included in the log information.

For example, the BD creation unit 312 calculates a statistical value such as an average value, a maximum value, or a minimum value for each smoking area. In this example, the BD creation unit 312 uses the position information included in the log information. For example, the BD creation unit 312 calculates a statistical value such as an average value, a maximum value, or a minimum value for each smoking area in each time zone. In this example, the BD creation unit 312 uses the time information and the position information included in the log information. Not limited to the above, the BD creation unit 312 may calculate a statistical value by arbitrarily combining data included in the log information. The BD creation unit 312 creates base data including the various statistical values described above. Furthermore, the information processing server 3 may acquire the basic data of the user and create the base data by measuring the biometric data of the user at the time of initial use or of registering the inhaling device 1, or by measuring the biometric data of the user for a certain period such as a week.

According to this example, the information processing server 3 can compare one or more index values included in the latest log information with various thresholds. Thus, the information processing server 3 can determine the stress state of the user from various perspectives. Further, the information processing server 3 can effectively determine the stress state of the user.

The storage control unit 313 performs control so that the base data created by the base data creation unit 312 is stored in the base data DB 322 (step S3). In step S3, for example, the storage control unit 313 accordingly adds the base data created by the base data creation unit 312 to the base data DB 322.

FIG. 11 is a flowchart showing an example of processing a recommendation by the information processing server according to the embodiment of the present invention.

The processing procedure described below is merely an example, and each act of processing may be changed as much as possibility allows. In addition, in the processing procedure described below, steps can be omitted, replaced, and added as appropriate according to the embodiment.

In order to simplify the description, the inhaling devices 1a and the user terminal 2a will be described below as examples.

The acquisition unit 311 acquires various data (step S11). In step S11, for example, the acquisition unit 311 acquires, via the first communication unit 33, biometric data measured by the inhaling device 1a based on activation of the inhaling device 1a. Here, the first communication unit 33 receives the biometric data measured by the inhaling device 1a via the first network. The first network is a mesh network including a plurality of inhaling devices 1 including the inhaling device 1a. The acquisition unit 311 acquires one or more index values based on the biometric data, and acquires one or more index values based on the biometric data. The acquisition unit 311 sequentially adds the log information including one or more index values to the log information DB 321 every time the predetermined period has passed in a time series. The acquisition unit 311 stores, in the log information, the time at which the biometric data is acquired. The acquisition unit 311 acquires various types of information included in the user data from the user terminal 2a via the second communication unit 34. For example, the acquisition unit 311 acquires position information from the user terminal 2a via the second communication unit 34. Here, the second communication unit 34 receives the position information from the user terminal 2a via the second network. The acquisition unit 311 acquires user information from the user terminal 2a via the second communication unit 34. Here, the second communication unit 34 receives the user information from the user terminals 2a via the second network. The acquisition unit 311 may separately acquire, from the user terminal 2a, various types of information described as may be included in the user data. The acquisition unit 311 accordingly adds the position information, the user information, and the like to the log information DB 321 as needed.

The comparison unit 314 compares at least one index value acquired by the acquisition unit 311 with the threshold value (step S12). In step S12, for example, the comparison unit 314 compares the at least one index value acquired by the acquisition unit 311 and included in the latest log information with the threshold value. The comparison unit 314 may compare at least one index value included in the latest log information with the threshold value every time the latest log information is updated. The threshold value corresponds to an arbitrary statistical value included in the base data stored in the base data DB 322. The threshold value may be a value higher than the arbitrary statistical value by a predetermined value. For example, the comparison unit 314 compares a "pulse rate" with a threshold value corresponding to a statistical value of an "average value of past logs" of pulse rates associated with the user ID of the user terminal 2a included in the base data. The comparison unit 314 may compare a stress level based on at least one index value acquired by the acquisition unit 311 with the stress level based on the statistical value serving as the threshold value.

The comparison unit 314 may compare, for each index, at least one index value with a threshold value corresponding to a statistical value associated with the user of the user terminal 2a. The comparison unit 314 may compare, for each index, at least one index value with a threshold value corresponding to a statistical value of all users within a plurality of users. The comparison unit 314 may compare, for each index, at least one index value with a threshold value corresponding to a statistical value for each group obtained by grouping according to an attribute.

The comparison unit 314 may compare, for each index, at least one index value with a plurality of threshold values corresponding to a plurality of statistical values included in the base data. For example, the comparison unit 314 compares for each index, at least one index value with threshold values respectively corresponding to the statistical values of the average value, the maximum value, and the minimum value. The comparison unit 314 may compare at least one index value included in the latest log information and acquired by the acquisition unit 311 with a threshold value corresponding to a statistical value under the same condition as the acquisition condition of the index value. For example, in consideration of at least one of the time information and the position information included in the log information, the comparison unit 314 uses a threshold value corresponding to a statistical value in consideration of at least one of the time zone and the smoking area.

The creation unit 315 creates stress state information based on at least one index value based on the comparison result of the comparison unit 314 (step S13). In step S13, for example, the creation unit 315 creates stress state information indicating a result of comparison between one index value and a threshold value as a stress state. The creation unit 315 records the created stress state information in the stress state information DB 323.

In a case where the index value is compared with one threshold value, the creation unit 315 determines the stress state based on the comparison result as exemplified below. If the index value is equal to or greater than the threshold value, the creation unit 315 may determine that the stress state is "high stress". Alternatively, if the index value is equal to or greater than the threshold value, the creation unit 315 may determine that the stress state is "medium stress" or "high stress" in accordance with the degree of divergence of the index value from the threshold value. If the index value is less than the threshold value, the creation unit 315 may determine that the stress state is "low stress".

In a case where the index value is compared with the threshold values respectively corresponding to the average value, the maximum value, and the minimum value, the creation unit 315 determines the stress state based on the comparison results as exemplified below. If the index value is less than the minimum value, the creation unit 315 may determine that the stress state is "stress level 0". If the index value is equal to or greater than the minimum value and less than the average value, the creation unit 315 may determine that the stress state is "stress level 1". If the index value is equal to or greater than the average value and less than the maximum value, the creation unit 315 may determine that the stress state is "stress level 2". If the index value is equal to or greater than the maximum value, the creation unit 3 15 may determine that the stress state is "stress level 3". The determination of the stress state is the same in an aspect in which a stress level based on at least one index value is compared with a stress level based on a statistical value serving as a threshold value. The creation unit 315 creates information indicating the determined stress state as stress state information. The creation unit 315 records the created stress state information in the stress state information DB 323.

In addition, the creation unit 315 may create, as the stress state information, a graph in which the relative position of the current index value of the user is superimposed on the distribution of index values. For example, in a case where the index value is compared with the statistical value of the user, the creation unit 315 creates, as the stress state information, a graph in which the relative position of the current index value of the user is superimposed on the distribution of the index values of the user. In a case where the index value is compared with the statistical value of the user, the creation unit 315 may create, as the stress state information, a graph in which the relative position of the stress level based on the current index value of the user is superimposed on the distribution of the stress levels based on the index value of the user. The creation unit 315 records the created stress state information in the stress state information DB 323.

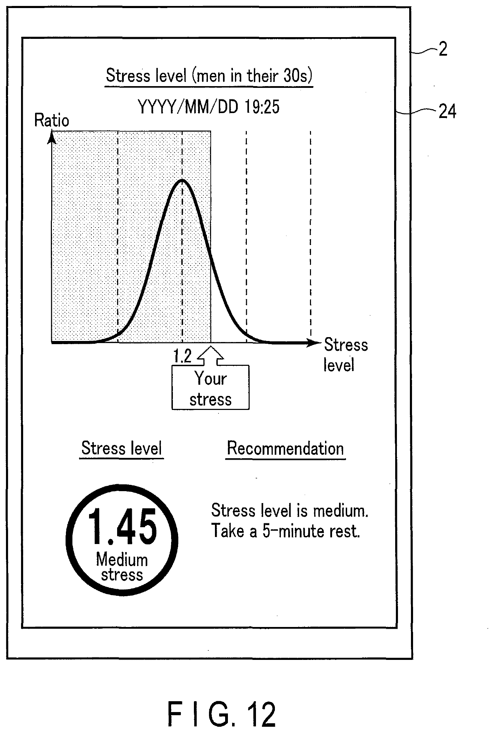

For example, in a case where the index value is compared with the statistical value of a group, the creation unit 315 creates, as the stress state information, a graph in which the relative position of the current index value of the user is superimposed on the distribution of the index values of the group. In a case where the index value is compared with the statistical value of a group, the creation unit 315 may create, as the stress state information, a graph in which the relative position of the stress level based on the current index value of the user is superimposed on the distribution of the stress levels based on the index values of the group. The creation unit 315 records the created stress state information in the stress state information DB 323. FIG. 12 is a diagram showing a display example of the display unit 24 of the user terminal 2a in a case where the current index value of the user is compared with the statistical value of "men in their 30s". According to FIG. 12, a graph showing a distribution of stress states of men in their 30s is displayed on the display unit 24 of the user terminal 2a. In the graph of the distribution of the stress states, the vertical axis represents the ratio of the number of persons, the horizontal axis represents the stress level, and the ratio of the number of persons corresponding to each stress level to the total number of persons in a group of men in their 30s is displayed as a distribution diagram. In the example of FIG. 12, the current stress level of the user is superimposed as "your stress" on the distribution diagram of the stress states of men in their 30s. The current stress level is displayed on the display unit 24 of the user terminal 2a. In the example of FIG. 12, "1.45" is displayed as the current stress level, and "medium stress" is displayed as the stress state. The current stress level is a stress level based on the current index value of the user. In this example, the average value of the stress levels of the group of men in their 30s is "1.2". The average value of the stress levels of the group of men in their 30s is a stress level based on the average value of the group of men in their 30s serving as a statistical value. The average value of the stress levels of the group of men in their 30s is clearly shown in the graph. Further, a recommendation based on the stress state information selected based on the current stress level is displayed on the display unit 24. In the example of FIG. 12, as the recommendation based on the stress state information, "The stress level is medium. Take a 5-minute rest." is displayed.

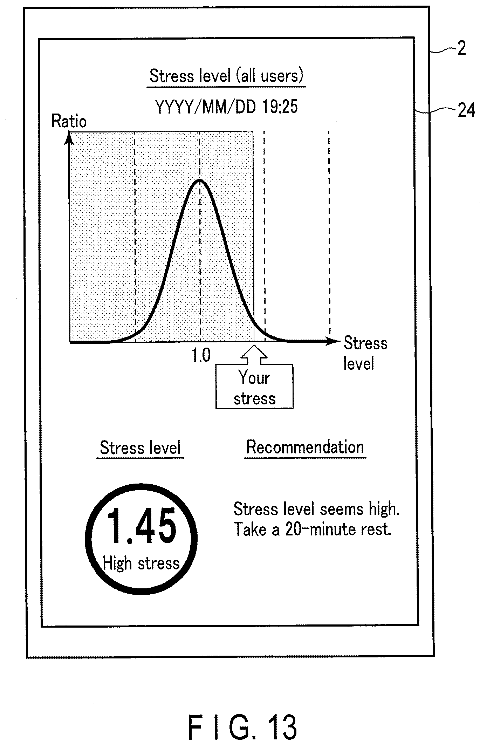

For example, in a case where the index value is compared with the statistical value of all users within the plurality of users, the creation unit 315 creates, as the stress state information, a graph in which the relative position of the current index value of the user is superimposed on the distribution of the index values for all users. In a case where the index value is compared with the statistical value of all users within the plurality of users, the creation unit 315 may create, as the stress state information, a graph in which the relative position of the current index value of the user is superimposed on the distribution of the index values for all users. The creation unit 315 records the created stress state information in the stress state information DB 323. FIG. 13 is a diagram showing a display example of the display unit 24 of the user terminal 2a in a case where the current index value of the user is compared with the statistical value for all users within the plurality of users. According to FIG. 13, a graph showing a distribution of stress states for all users is displayed on the display unit 24 of the user terminal 2a. In the graph of the distribution of the stress states, the vertical axis represents the ratio of the number of persons, the horizontal axis represents the stress level, and the ratio of the number of persons corresponding to each stress level to the total number for all users within the plurality of users is displayed as a distribution diagram. In the example of FIG. 13, the current stress level of the user is superimposed as "your stress" on the distribution diagram of the stress states for all users. The current stress level is displayed on the display unit 24 of the user terminal 2a. In the example of FIG. 13, "1.45" is displayed as the current stress level, and "high stress" is displayed as the stress state. The current stress level is a stress level based on the current index value of the user. In this example, the average value of the stress levels for all users within the plurality of users is "1.0". The average value of the stress levels for users within the plurality of users is a stress level based on the average value for all users within the plurality of users serving as a statistical value. The average value of the stress levels for all users within the plurality of users is clearly shown in the graph. Further, a recommendation based on the stress state information selected based on the current stress level is displayed on the display unit 24 of the user terminal 2a. In the example of FIG. 13, as the recommendation based on the stress state information, "The stress level seems high. Take a 20-minute rest." is displayed.

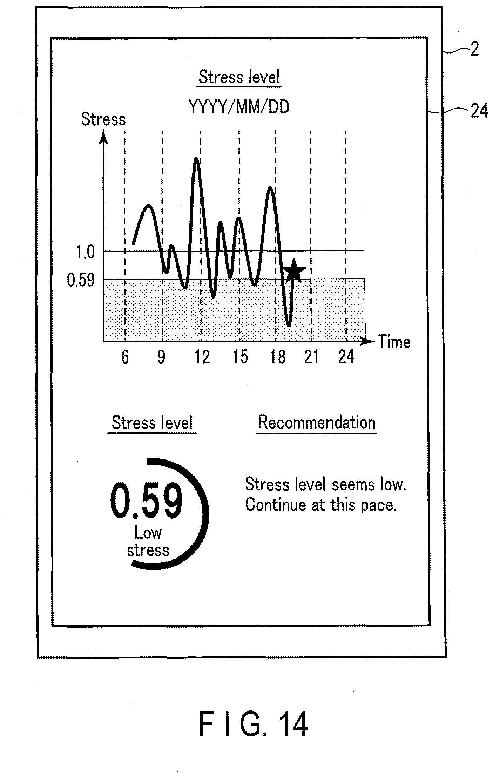

In addition, the creation unit 315 may create, as the stress state information, a graph in which the current index value is clearly indicated in the transition of the index value of the user within a given day. The creation unit 315 may create, as the stress state information, a graph in which the user stress state transition within a given day is clearly indicated. The stress state transition is a transition of the stress level based on the index value. The creation unit 315 records the created stress state information in the stress state information DB 323. FIG. 14 is a diagram showing a display example of the display unit 24 of the user terminal 2a for a graph that clearly shows a user stress state transition within a given day. According to FIG. 14, a graph showing the user stress state transition within a given day is displayed on the display unit 24 of the user terminal 2a. In the graph of the stress state transition, the vertical axis represents the stress level, the horizontal axis represents the time, and the stress level corresponding to each time is displayed as a smooth line graph. In the example of FIG. 14, the current stress level of the user is superimposed on the graph with a star sign. The current stress level is displayed on the display unit 24 of the user terminal 2a. In the example of FIG. 14, "0.59" is displayed as the current stress level, and "low stress" is displayed as the stress state. The current stress level is a stress level based on the current index value of the user. In this example, the average value of the stress levels of the user in the past day is "1.0". The average value of the past stress levels of the user is an average value of stress levels based on the past index values of the user. The average value of the past stress levels of the user is clearly shown in the graph. Further, a recommendation based on the stress state information selected based on the current stress level is displayed on the display unit 24 of the user terminal 2a. In the example of FIG. 14, as the recommendation based on the stress state information, "The stress level seems low. Continue at this pace." is displayed.

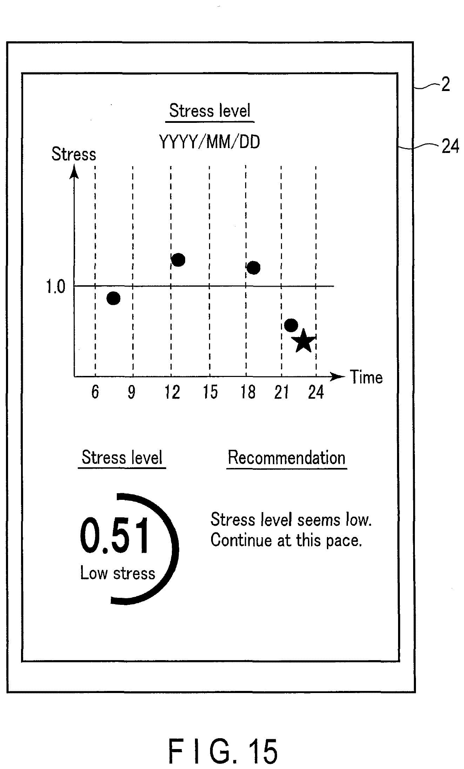

In addition, the creation unit 315 may create, as the stress state information, a graph in which the current index value is clearly indicated in the transition of the index value of the user at a predetermined timing within a given day. The creation unit 315 may create, as the stress state information, a graph that clearly indicates the user stress state transition at a predetermined timing within a given day. For example, the predetermined timing is a time of smoking. The time of smoking may be before smoking, after smoking, or both. The creation unit 315 records the created stress state information in the stress state information DB 323. FIG. 15 is a diagram showing a display example of the display unit 24 of the user terminal 2a for a graph that clearly shows a transition of the user stress state at the times of smoking within a given day. In the graph of stress state transition, the vertical axis represents the stress level, the horizontal axis represents the time, and the stress level corresponding to each timing of smoking is displayed as a plot diagram. In the example of FIG. 15, the current stress level of the user is plotted on the graph with a star sign. The current stress level is displayed on the display unit 24 of the user terminal 2a. In the example of FIG. 15, "0.51" is displayed as the current stress level, and "low stress" is displayed as the stress state. The current stress level is a stress level based on the current index value of the user. In this example, the average value of all of the past stress levels of the user is "1.0". The average value of the past stress levels of the user is an average value of stress levels based on the past index values of the user. The average value of the past stress levels of the user is clearly shown in the graph. Further, a recommendation based on the stress state information selected based on the current stress level is displayed on the display unit 24 of the user terminal 2a. In the example of FIG. 15, as the recommendation based on the stress state information, "The stress level seems low. Continue at this pace." is displayed.

The stress state information is not limited to text and graphs, and may be in any format. The determination of the stress state can be achieved using known techniques. According to this example, the information processing server 3 can provide the user stress state in an easily visible format such as text or a graph. The information processing server 3 can recommend the user to take a measure for stress relief.

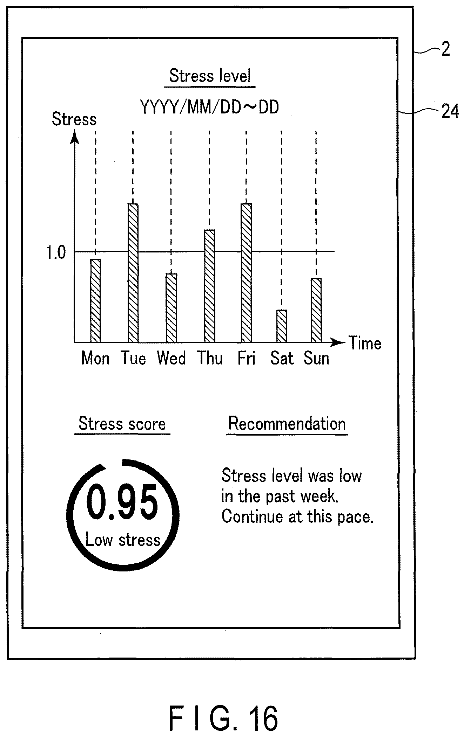

In addition, the creation unit 315 may create, as the stress state information, a graph in which the user stress state transition during a predetermined period is clearly indicated. The predetermined period of time may be, for example, one day, one week, one month, or the like. FIG. 16 is a diagram showing a display example of the display unit 24 of the user terminal 2a in a case where the predetermined period is one week. According to FIG. 16, a graph showing a transition of the user stress state within one week is displayed on the display unit 24 of the user terminal 2a. In the graph of the stress state transition, the vertical axis represents the stress level, the horizontal axis represents the time (the day of the week), and the stress level corresponding to each day of the week is displayed as a bar graph. A stress score indicating an average value of the stress levels in a given week is displayed on the display unit 24 of the user terminal 2a. In the example of FIG. 16, "0.95" is displayed as the stress score, and "low stress" is displayed as the stress state. In this example, the average value of all of the past stress levels of the user is "1.0". The average value of the past stress levels of the user is clearly shown in the graph. Furthermore, a recommendation selected based on the stress score for one week is displayed on the display unit 24 of the user terminal 2a. In the example of FIG. 16, as the recommendation, "The stress level was low in the past week. Continue at this pace." is displayed.

As a result of comparing at least one index value with the threshold values in step S12, if the comparison unit 314 determines that the at least one index value is equal to or greater than at least one threshold value (step S14: YES), the process transitions from step S14 to step S15. As a result of comparing at least one index value with the threshold values in step S12, if the comparison unit 314 determines that no index value is equal to or greater than any threshold value (step S14: NO), the process transitions from step S14 to step S16.

The selection unit 316 selects at least one recommendation in response to at least one index value being equal to or greater than the threshold value (step S15). In step S15, for example, the selection unit 316 selects at least one recommendation stored in the recommendation DB 324. The selection unit 316 selects at least one recommendation in consideration of the user information acquired by the acquisition unit 311.

The selection unit 316 selects a recommendation based on the stress state information as a recommendation included in the at least one recommendation. For example, in a case where the stress state is "medium stress" or higher, or "stress level 1" or higher, the selection unit 316 selects a recommendation based on the stress state information for stress relief from the recommendation DB 324. For example, the recommendation based on the stress state information includes a recommendation for recommending a rest, a recommendation for a flavor for stress relief, a recommendation for food or drink such as coffee or soft drink, a recommendation for a browsable video or moving image, a recommendation for a game, and the like. In a specific example, the recommendation based on the stress state information is the text of a comment such as "Take a rest for 5 minutes" or "XX is recommended". The recommendation based on the stress state information may be a uniform resource locator (URL) of a service in which users whose stress state is determined to be "medium stress" or higher or "stress level 1" or higher can communicate with each other. According to this example, the information processing server 3 can select a recommendation to recommend the user to take a measure for stress relief in accordance with the user stress state. The information processing server 3 can recommend the user to take a measure for stress relief based on the selected recommendation.

In addition, the selection unit 316 may select a recommendation based on the stress state information in consideration of the user information acquired by the acquisition unit 311. For example, in a case where the user information includes user preference information, the selection unit 316 may select a recommendation of a flavor, food or drink such as coffee or confectionery, a browsable video or moving image, a game, or the like in consideration of the preference information. According to this example, the information processing server 3 can select a recommendation in consideration of the user information such as the user preference in accordance with the user stress state. The information processing server 3 can recommend each user to take a more appropriate measure for stress relief based on the selected recommendation.

In addition, the selection unit 316 may select a recommendation based on the stress state information in consideration of the type information acquired by the acquisition unit 311. For example, in a case where the user data includes type information, the selection unit 316 may select a recommendation of a flavor according to the type of the inhaling device 1a in consideration of the type information. According to this example, the information processing server 3 can select a recommendation in consideration of the type information of the inhaling device 1a in accordance with the user stress state. The information processing server 3 can recommend each user to take a more appropriate measure for stress relief based on the selected recommendation.

The selection unit 316 selects a recommendation based on information differing from the stress state information as a recommendation included in the at least one recommendation. For example, the information differing from the stress state information is position information on the user terminal 2a acquired by the acquisition unit 311. In this example, as exemplified below, the selection unit 316 selects a rest facility as a recommendation included in the at least one recommendation based on the position information on the user terminal 2a. The selection unit 316 acquires the position information on the user terminal 2a included in the latest log information. The selection unit 316 acquires the position information on rest facilities stored in the recommendation DB 324 among the position information on the respective facilities included in the map information DB 325. The selection unit 316 compares the position information on the user terminal 2a with the position information on the rest facilities. The selection unit 316 selects one or more rest facilities near the current location of the user. The number of rest facilities selected by the selection unit 316 can be set as appropriate. The range centered on the current location of the user for the selection unit 316 to select rest facilities can be set as appropriate. The selection unit 316 selects the selected one or more rest facilities as recommendations. According to this example, the information processing server 3 can select a rest facility near the current location of the user as a recommendation in accordance with the user stress state. The information processing server 3 can recommend the user to a take a measure for stress relief based on the selected recommendation, such as visiting a nearby rest facility.

In addition, the selection unit 316 may select a recommendation based on the position information in consideration of the user information acquired by the acquisition unit 311. For example, in a case where the user information includes user preference information, the selection unit 316 compares the user preference information with attribute information of the respective facilities included in the map information DB 325. The selection unit 316 may select, as a recommendation, a rest facility that matches the user preference from among one or more rest facilities near the current user location. For example, in a case where the preference information includes information indicating that the user likes coffee, the selection unit 316 may select a coffee shop near the current location of the user as a recommendation. According to this example, the information processing server 3 can select a recommendation in consideration of the user information, such as the user preference, and the position information in accordance with the user stress state. Based on the selected recommendation, the information processing server 3 can recommend a rest facility that is close to the current location of the user and suits the preference of the user. The information processing server 3 can recommend each user to take a more appropriate measure for stress relief.

The recommendation based on information differing from the stress state information may be information such as a positive word, the words of an eminent person, fortune-telling, news, or the like. The recommendation based on information differing from the stress state information may be the provision of points, coins, game items, or the like. In a case where the user terminal 2a is a device that emits sound, the recommendation based on information differing from the stress state information may be predetermined audio information such as a person's laughter. In a case where the user terminal 2a is a device that emits light, the recommendation based on information differing from the stress state information may be a predetermined light emission pattern. In a case where the user terminal 2a is a vibratory device, the recommendation based on information differing from the stress state information may be a predetermined vibration pattern. According to this example, the information processing server 3 can select various recommendations in accordance with the user stress state. The information processing server 3 can recommend the user to take measures for relieving various stresses based on the selected recommendations. The selection of recommendations may be performed using known machine learning techniques.

As a recommendation included in the at least one recommendation, the selection unit 316 may independently select either or both of: the recommendation based on the stress state information and the recommendation based on information differing from the stress state information.

The output unit 317 outputs at least the stress state information (step S16). In step S16, for example, in a case where the selection unit 316 does not select at least one recommendation, the output unit 317 outputs the stress state information to the user terminal 2a via the second communication unit 34. In this example, the second communication unit 34 transmits the stress state information to the user terminal 2a via the second network. For example, in a case where the selection unit 316 selects at least one recommendation, the output unit 317 outputs the stress state information and the at least one recommendation to the user terminal 2a via the second communication unit 34. In this example, the second communication unit 34 transmits the stress state information and the at least one recommendation to the user terminal 2a via the second network.

In a case where the output unit 317 outputs the stress state information created by the creation unit 315 in step S13, the user terminal 2a displays the stress state information on the display unit 24 based on receipt of the stress state information. The display of the stress state information may be, but is not limited to, text display, graph display, or the like, and may be of any display mode. According to this example, the information processing server 3 can provide the user stress state in an easily visible format. The information processing server 3 can recommend the user to take a measure for stress relief.

In a case where the output unit 317 outputs one or more recommendations selected by the selection unit 316 in step S15, the user terminal 2a displays the one or more recommendations on the display unit 24 based on receipt of the one or more recommendations.

In a case where the selection unit 316 selects the recommendation based on the stress state information, the output unit 317 outputs the recommendation based on the stress state information. Based on receipt of the recommendation based on the stress state information, the user terminal 2a displays the recommendation on the basis of the stress state information on the display unit 24.

In a case where the selection unit 316 selects the recommendation based on the information differing from the stress state information, the output unit 317 outputs the recommendation based on the information differing from the stress state information. Based on receipt of the recommendation based on the information differing from the stress state information, the user terminal 2a displays the recommendation based on the information differing from the stress state information on the display unit 24. For example, in a case where the selection unit 316 selects a rest facility, the output unit 317 outputs a map on which the rest facility is superimposed. In this example, the second communication unit 34 transmits the map on which the rest facility is superimposed to the user terminal 2a via the second network. The user terminal 2a displays the map on which the rest facility is superimposed on the display unit 24 based on the receipt of the map on which the rest facility is superimposed.

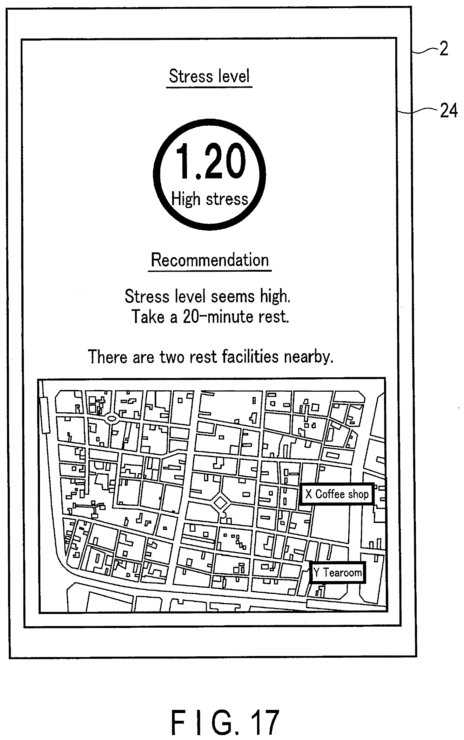

FIG. 17 is a diagram illustrating a display example of the display unit 24 of the user terminal 2a in a case where a rest facility is selected as a recommendation. In the example of FIG. 17, "1.20" is displayed as the current stress level, and "high stress" is displayed as the stress state. The current stress level is a stress level based on the current index value of the user. Further, a recommendation based on the stress state information selected based on the current stress level is displayed on the display unit 24 of the user terminal 2a. In the example of FIG. 17, as the recommendation based on the stress state information, "The stress level seems high. Take a 20-minute rest." is displayed. Further, the display unit 24 of the user terminal 2a displays the rest facility selected by the selection unit 316, based on the position information on the user terminal 2a, so as to be superimposed on the map around the current location. In the example of FIG. 17, a coffee shop and a tearoom are displayed on the map as rest facilities close to the current location of the user. In the example of FIG. 17, as the recommendation based on information differing from the stress state information, "There are two rest facilities nearby." is displayed.

The display of the at least one recommendation may be, but is not limited to text display, map display, or the like, and may be of any display mode. According to this example, the information processing server 3 can provide the recommendation in an easily visible format. The information processing server 3 can recommend the user to take a measure for stress relief.

The at least one recommendation may be displayed either on the same screen as the stress state information or on another screen. For example, the recommendation based on the stress state information may be displayed either on the same screen as the stress state information or on another screen. For example, the recommendation based on the information differing from the stress state information may be displayed either on the same screen as the stress state information or on another screen. For example, the recommendation based on the stress state information may be displayed either on the same screen as the recommendation based on information differing from the stress state information, or on another screen.

The output unit 317 may output, to the user terminal 2a, an appropriate display instruction in accordance with the recommendation selected by the selection unit 316. For example, in a case where points, coins, or the like are selected as a recommendation, the output unit 317 may output an instruction to display the number of points or coins to be given to the user terminal 2a. For example, in a case where a sound, a light emission pattern, or a vibration pattern is selected as a recommendation, the output unit 317 may output an instruction to provide them to the user terminal 2a. For example, in a case where a communication service is selected as a recommendation, the output unit 317 may output an instruction to display the URL of the service to the user terminal 2a. According to this example, the information processing server 3 can provide various recommendations to the user. The information processing server 3 can recommend the user to take measures for relieving various stresses.

In a case where the inhaling device 1a includes a display unit, the display unit of the inhaling device 1a may control display of the stress state information and the at least one recommendation. In this example, the output unit 317 outputs, to the user terminal 2a via the second communication unit 34, the stress state information, the at least one recommendation, and an instruction to transfer them to the inhaling device 1a. In this case, the display mode may be the same as or different from that of the user terminal 2a. For example, the display for the inhaling device 1a may be a display of abbreviations, icons, or the like, indicating the stress state information, or abbreviations, icons, or the like, indicating the content of the recommendation. According to this example, the information processing server 3 can provide the stress state information and the recommendation at the inhaling device 1a. The information processing server 3 can increase the visibility to the user and more efficiently recommend the user to take a measure for stress relief.

In step S14, if the comparison unit 314 determines that at least one index value is equal to or greater than at least one threshold value, the output unit 317 may transmit a control command. The transmission unit 318 outputs the control command for controlling the inhaling device 1a to the user terminal 2a via the second communication unit 34 in response to at least one index value being equal to or greater than the threshold value. In this example, the second communication unit 34 transmits the control command to the user terminal 2a via the second network in response to at least one index value being equal to or greater than the threshold value. Based on receipt of the control command, the user terminal 2a forwards the control command to the inhaling device 1a. The inhaling device 1a controls the heating temperature, the smoke amount, etc. using any known technique based on the received control commands. According to this example, if it is determined that the stress is high, the information processing server 3 can change the heating temperature, the smoke amount, and the like of the inhaling device 1a. The information processing server 3 can control the inhaling state of the user and support relief of the stress state.

According to the embodiment, the inhaling device 1 can transmit the biometric data to the information processing server 3 without going through the user terminal 2. In addition, even in an environment in which the inhaling device 1 cannot be directly connected to the base station 4, it is possible to reliably transmit the biometric data to the information processing server 3 via the mesh network including a plurality of inhaling devices 1.

### <<Modification>>

The embodiment may include the following modification.

The selection unit 316 may select a recommendation based on the stress state information with the length of the rest time being changed according to the degree of divergence of at least one index value from the threshold value. The selection unit 316 may set the rest time to be longer as the degree of divergence increases. According to this example, the information processing server 3 can recommend the user to take an appropriate measure for stress relief according to the degree of the stress state.

Further, in a case where the comparison unit 314 compares at least one index value with a threshold value corresponding to the maximum value, the selection unit 316 may select at least one recommendation according to the magnitude of the at least one index value as exemplified below. For example, in response to at least one index value being equal to or greater than the maximum value, the selection unit 316 selects a recommendation based on stress state information indicating that the stress state is very high. The recommendation based on the stress state information is a warning that strongly recommends stress relief, a recommendation that recommends a rest for a length of time (for example, 20 minutes) longer than a normal length of time (for example, 5 minutes), or the like. In this example, the output unit 317 may output a control command for prohibiting the use of the inhaling device 1 until at least one index value becomes less than the maximum value. The at least one index value to be compared by the comparison unit 314 may be an index value before smoking, an index value during smoking, or an index value after smoking.

Further, in a case where the comparison unit 314 compares at least one index value with a threshold value corresponding to the minimum value, the selection unit 316 may select at least one recommendation according to the magnitude of the at least one index value as exemplified below. For example, in response to at least one index value being less than the minimum value, the selection unit 316 selects a recommendation based on stress state information indicating an abnormal value. The recommendation based on the stress state information is a warning that strongly recommends a rest, a recommendation that recommends a rest for a length of time (for example, 20 minutes) longer than a normal length of time (for example, 5 minutes), or the like. The recommendation based on the stress state information may be a recommendation for recommending a check of the state of the inhaling device 1 in order to re-measure the biometric data in the inhaling device 1. This is because there is a possibility that the inhaling device 1 does not accurately measure biometric data. In this example, the output unit 317 may output control command for prohibiting the use of the inhaling device 1 until at least one index value becomes equal to or greater than the minimum value. The at least one index value to be compared by the comparison unit 314 may be an index value before smoking, an index value during smoking, or an index value after smoking.

Further, in a case where the comparison unit 314 compares at least one index value with threshold values respectively corresponding to the average value, the maximum value, and the minimum value, the selection unit 316 may select at least one recommendation according to the magnitude of the at least one index value as exemplified below. For example, the selection unit 316 selects at least one recommendation in response to the at least one index value being closer to the maximum value than the average value. This is because the comparison unit 314 determines that the user stress state is high. For example, the selection unit 316 omits selection of at least one recommendation in response to the at least one index value being closer to the minimum value than the average value. This is because the comparison unit 314 determines that the user stress state is not high.

Furthermore, in a case where the comparison unit 314 compares at least one index value related to salivary components with a threshold value, the selection unit 316 may select a recommendation for an oral cavity state determinable from the measured salivary components. For example, the selection unit 316 may select a recommendation related to breath odor and periodontal diseases.

Furthermore, when acquiring the biometric data measured by the inhaling device 1, the information processing server 3 may give points, coins, items, or the like to the user terminal 2. In addition, the information processing server 3 may change points or the like to be given in accordance with the degree of the stress state. According to this example, the information processing server 3 can be expected to increase opportunities for providing biometric data by the user, and can effectively execute creation of stress state information.

Furthermore, the information processing server 3 may select a recommendation in consideration of user schedule information by cooperating with a scheduler of the user terminal 2. According to this example, the information processing server 3 can recommend the user to take a measure for stress relief in accordance with the user schedule information.

Next, a modification of the mesh network of the embodiment will be described.

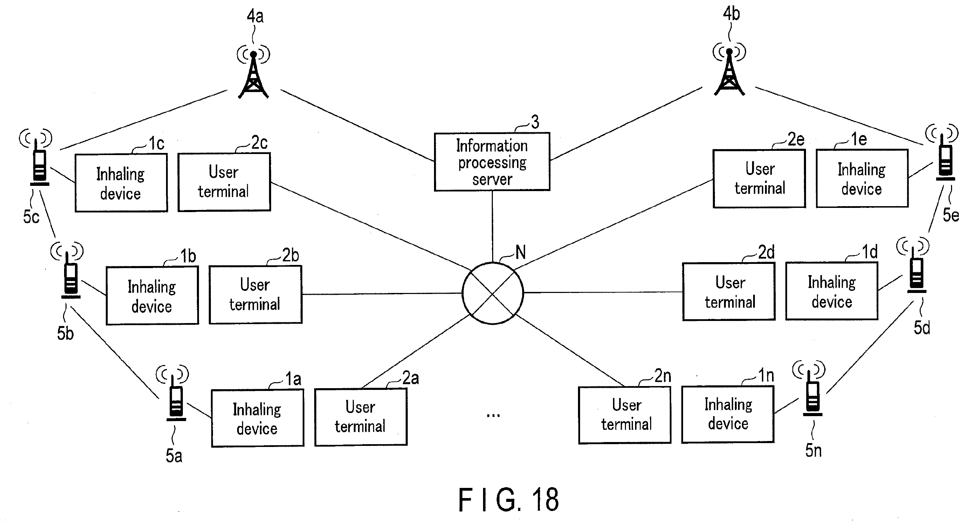

FIG. 18 is a block diagram schematically showing a modification of a configuration of the information distribution system according to the embodiment of the present invention.

The information distribution system 10 further includes six small base stations 5a to 5c and 5n in addition to the configuration of FIG. 1. When the six small base stations are not distinguished from one another, each one of them will be simply referred to as a "small base station 5", with part of the reference symbol omitted. The number of the small base stations 5 is not limited to six.

The small base station 5 includes a communication interface compatible with the LPWA communication standard. The plurality of small base stations 5 construct a mesh network and can communicate with one another. The small base stations 5 are disposed in smoking areas or the like. The smoking areas include indoor or outdoor smoking spaces, smoking-allowed restaurants, or the like.

The small base station 5 transmits the biometric data to the information processing server 3 via the first network. The first network is a mesh network including a plurality of small base stations 5 capable of communicating with the inhaling device 1. The communication interface of the small base stations 5 is not limited to the LPWA communication standard, and may be any communication interface compatible with a communication standard capable of constructing a mesh network, such as a local 5G.

The inhaling device 1 includes a communication interface that enables communications compatible with the communication standard of a third network. The third network is a network different from the first network and the second network. For example, the third network is short-range wireless communication such as Bluetooth (registered trademark), BLE (Bluetooth Low Energy), Wi-Fi (registered trademark), or NFC (Near Field Communication) communication. The transmission and reception of data between the inhaling device 1 and the small base station 5 are not limited to these, and may be executed by any communication.

Taking the inhaling device 100A as an example, the communication unit 115A of the inhaling device 100A is a communication interface that enables communications compatible with the communication standard of the third network. The communication unit 115A enables communications between the inhaling device 1 and the user terminal 2 via the third network. The communication unit 115A transmits, via the third network, the biometric data to at least one of the plurality of small base stations 5 included in the mesh network. The communication unit 115B of the inhaling device 100B may be configured in the same manner as the communication unit 115A.

The small base station 5 transmits the biometric data received from the inhaling device 1 to the base station 4 via the first network toward the information processing server 3. The base station 4 transmits the biometric data received from the small base station 5 to the information processing server 3.

According to the modification, even in a case where the inhaling device 1 does not include a communication interface compatible with an LPWA or the like, the inhaling device 1 can communicate with the small base station 5 disposed in a smoking area or the like. Therefore, the inhaling device 1 can transmit the biometric data to the information processing server 3 via the mesh network including the plurality of small base stations 5.

The present invention is not limited to the above-described embodiments, and can be modified in various manners in practice during implementation without departing from the gist of the invention. Moreover, the embodiments can be suitably combined; in that case, the combined advantages are obtained. Furthermore, the above-described embodiments include various inventions, and various inventions can be extracted by a combination selected from structural elements disclosed herein. For example, if the object of the invention is achieved and the advantages of the invention are attained even after some of the structural elements disclosed in connection with the embodiments are deleted, the structure made up of the resultant structural elements can be extracted as an invention.

### REFERENCE SIGNS LIST

1 ... inhaling device, 1a-1n ... inhaling device, 2 ... user terminal, 2a-2n ... user terminal, 3 ... information processing server, 4 ... base station, 4a, 4b ... base station, 5 ... small base station, 5a-5e, 5n ... small base station, 10 ... information distribution system, 21 ... control unit, 22 ... storage unit, 23 ... input unit, 24 ... display unit, 25 ... communication unit, 26 ... detection unit, 31 ... control unit, 32 ... storage unit, 33 ... first communication unit, 34 ... second communication unit, 100A ... inhaling device, 100B ... inhaling device, 110 ... power-supply unit, 111A ... power supply, 111B ... power supply, 112A ... sensor unit, 112B ... sensor unit, 113A ... notification unit, 113B ... notification unit, 114A ... storage unit, 114B ... storage unit, 115A ... communication unit, 115B ... communication unit, 116A ... control unit, 116B ... control unit, 120 ... cartridge, 121A ... heating unit, 121B ... heating unit, 122 ... liquid guide unit, 123 ... liquid storage unit, 124 ... mouthpiece, 130 ... flavor-imparting cartridge, 131 ... flavor source, 140 ... holding part, 141 ... internal space, 142 ... opening, 143 ... bottom portion, 144 ... heat-insulating part, 150 ... stick-shaped base material, 151 ... base material portion, 152 ... suction portion, 180 ... airflow path, 181 ... air inflow hole, 182 ... air outflow hole, 190 ... arrow, 311 ... acquisition unit, 312 ... BD creation unit, 313 ... storage control unit, 314 ... comparison unit, 315 ... creation unit, 316 ... selection unit, 317 ... output unit, 321 ... log information DB, 322 ... base data DB, 323 ... stress state information DB, 324 ... recommendation DB, 325 ... map information DB

## Claims

1. An information distribution system comprising a flavor inhaler and an information processing device,
the flavor inhaler comprising:
a sensor unit configured to measure biometric data; and
a communication unit configured to transmit, via a first network, the biometric data to the information processing device, and
the information processing device comprising:
a first communication unit configured to receive, via the first network, the biometric data measured by the flavor inhaler;
an acquisition unit configured to acquire at least one index value based on the biometric data;
a comparison unit configured to compare the at least one index value with a threshold value;
a creation unit configured to create, based on a comparison result of the comparison unit, stress state information based on the at least one index value;
a selection unit configured to select at least one recommendation according to the at least one index value; and
a second communication unit configured to transmit, to a user terminal via a second network different from the first network, the stress state information created by the creation unit and the at least one recommendation selected by the selection unit.

2. The information distribution system according to claim 1, wherein the first network is a mesh network including a plurality of flavor inhalers including the flavor inhaler.

3. The information distribution system according to claim 1, wherein the first network is a mesh network including a plurality of base stations communicable with the flavor inhaler.

4. The information distribution system according to claim 3, wherein the communication unit of the flavor inhaler is configured to transmit, via a third network, the biometric data to at least one of the plurality of base stations included in the mesh network.

5. The information distribution system according to any one of claims 1 to 4, wherein
the selection unit is configured to select a recommendation based on the stress state information as a recommendation included in the at least one recommendation, and
the second communication unit is configured to transmit the recommendation based on the stress state information selected by the selection unit.

6. The information distribution system according to any one of claims 1 to 5, wherein
the second communication unit is configured to receive position information on the user terminal via the second network,
the selection unit is configured to select a rest facility based on the position information as a recommendation included in the at least one recommendation, and
the second communication unit is configured to transmit, via the second network, a map on which the rest facility selected by the selection unit is superimposed.

7. The information distribution system according to any one of claims 1 to 6, wherein
the second communication unit is configured to receive user information via the second network, and
the selection unit is configured to select the at least one recommendation in consideration of the user information.

8. The information distribution system according to claim 5, wherein the recommendation based on the stress state information includes a message that recommends an action to reduce stress.

9. The information distribution system according to any one of claims 1 to 8, wherein the second communication unit is configured to transmit, via the second network, a control command for controlling the flavor inhaler according to the at least one index value.

10. An information processing device, comprising:
a first communication unit configured to receive, from a flavor inhaler via a first network, biometric data measured by the flavor inhaler;
an acquisition unit configured to acquire at least one index value based on the biometric data;
a comparison unit configured to compare the at least one index value with a threshold value;
a creation unit configured to create, based on a comparison result of the comparison unit, stress state information based on the at least one index value;
a selection unit configured to select at least one recommendation according to the at least one index value; and
a second communication unit configured to transmit, to a user terminal via a second network different from the first network, the stress state information created by the creation unit and the at least one recommendation selected by the selection unit.

11. An information distribution method in an information distribution system comprising a flavor inhaler and an information processing device, the method comprising:
by the flavor inhaler,
measuring biometric data; and
transmitting, via a first network, the biometric data to the information processing device, and
by the information processing device,
receiving, via the first network, the biometric data measured by the flavor inhaler;
acquiring at least one index value based on the biometric data;
comparing the at least one index value with a threshold value;
creating, based on a comparison result, stress state information based on the at least one index value;
selecting at least one recommendation according to the at least one index value; and
transmitting, to a user terminal via a second network different from the first network, the created stress state information and the selected at least one recommendation.
